Europäisches Patentamt

(19) European Patent Office

Office européen des brevets

(11) Publication number: **0 028 053**
**B1**

(12) **EUROPEAN PATENT SPECIFICATION**

(45) Date of publication of patent specification: **30.01.85**

(21) Application number: **80201036.3**

(22) Date of filing: **30.10.80**

(51) Int. Cl.⁴: **C 02 F 3/28, A 01 C 3/02,**
**C 12 M 1/00**

(54) **Closed apparatus for preparing biogas from manure.**

(30) Priority: **30.10.79 NL 7907970**
**02.04.80 NL 8001959**

(43) Date of publication of application:
**06.05.81 Bulletin 81/18**

(45) Publication of the grant of the patent:
**30.01.85 Bulletin 85/05**

(84) Designated Contracting States:
**AT BE CH DE FR GB IT LI LU NL SE**

(56) References cited:
**DE-A-1 459 472**
**DE-A-2 821 790**
**DE-C- 907 280**
**FR-A-1 474 824**
**US-A-2 605 014**
**US-A-3 535 236**
**US-A-4 060 175**

(73) Proprietor: **Paques B.V.**
**T. de Boerstraat 11**
**NL-8561 DT Balk (NL)**

(72) Inventor: **Vellinga, Sjoerd**
**De Terp 1**
**NL-8731 AX Wommels (NL)**

(74) Representative: **Urbanus, Henricus Maria, Ir.**
**et al**
**c/o Vereenigde Octrooibureaux Nieuwe**
**Parklaan 107**
**NL-2587 BP 's-Gravenhage (NL)**

Courier Press, Leamington Spa, England.

## Description

This invention relates to a closed apparatus for preparing biogas for manure, comprising a wall member (1), a manure fermenting section (2), means (4) for supplying manure to and means (5) for discharging fermented manure from said manure fermenting section (2), and means (9) for discharging the biogas prepared from the apparatus, and the apparatus further comprising means (7, 8) for heating and means (41) for stirring the manure both means being combined in a tubular assembly (37) through which a fluid can flow and which is double-walled over at least part of its length to allow circulation of a heating fluid between the double walls and through supply and discharge conduits (7, 8) connected thereto.

Such an apparatus is disclosed in German Patent 907,280 on the understanding, however, that the prior apparatus serves the purification of waste water. For this purpose the waste water is subjected in a central fermentation tank to an anaerobic digesting process leading to the decomposition of the organic material thereby generating gaseous and liquid material as well as partly causing mineralisation. This purifying process is promoted when the waste water to be purified in the central fermentation tank is agitated, for example by means of an agitating device or through circulation, and is heated. For this purpose the prior apparatus is provided with means, the working of which is such that with its help the circulation of the waste water and the heating of the waste water are combined. Said means comprise a tubular assembly being centrally and vertically positioned in the central fermentation tank, consisting of a tube being open at both ends and being double-walled, the room between both walls being connected via conduit-pipes to a heating member for heating of the heating fluidum.

The tube is also provided with a circulation member by means of which the waste water, which is to be purified, will pass the tube from bottom to top and at the same time maintaining a circulation stream in the waste water in the central fermenting tank.

It was shown in practice that the fermentation of manure is a process which is difficult to control, because manure is an inhomogeneous material, the composition of which is constantly varying. Compared to waste water manure, among others, has a far greater viscosity and specific weight while the variation of its composition, chemically as well as biochemically, is dependent on the animal's dietary. In practice one therefore has to contend with problems due to stratification of the manure.

It is an object of the invention to achieve a well controlled mixing of the manure in a manure fermenting tank while maintaining at the same time the heat exchange with the manure as constant as possible as well as maintaining an optimally even heat distribution in the manure for the purpose of a maximum recovery of the fermenting process, i.e. maximum biogas recovery.

The invention therefore, to achieve this object, is characterized in that the tubular assembly (37) is arranged so that it extends diagonally downward through the wall member (1) of the apparatus into the manure and a manure supply opening (40) is provided at a single-walled portion of the tubular assembly which is located between the double-walled portion (38) and the wall member of the apparatus, towards the upper end (39) of the tubular assembly and the stirring member, mounted on a shaft, is inserted through the upper end into the tubular assembly and is located below the manure supply opening (40).

The stirring member may be a propeller stirrer, which, secured to the shaft, is inserted into the tubular assembly from outside the silo, and via the shaft can be rotated by means, for example, of an electric motor. As a result, a circulation current will be generated between the bottom and top layers of the manure, whereby manure is "sucked" through the manure supply opening into the tubular assembly, and during the passage thereof is heated, and subsequently returned to the bulk of the manure.

By heating the manure as it flows along the heated surface, an optimum transfer of heat is obtained and the occurrence of depositions is prevented. Where deposits still occur they can easily be removed by removing the stirrer with the shaft and the electric motor axially from the tubular assembly, and scraping the deposits from the inner wall of the tubular assembly.

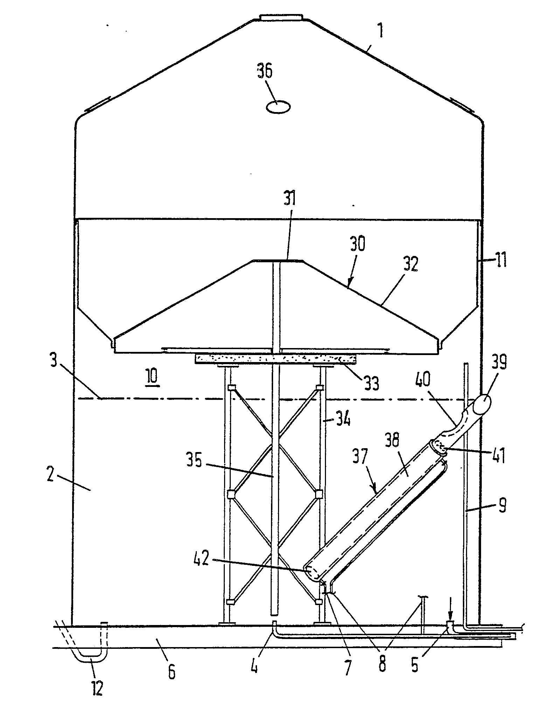

The apparatus according to the present invention will now be described, by way of example, with reference to the accompanying drawing, in which schematically in elevational view with the wall partly broken away there is shown apparatus according to the invention executed within a type of fodder silo much used on the farm with a gas buffer section comprising a flexible wall portion and a roof member which can be vertically displaced without guide means in a self-stabilizing manner, and in which the manure fermenting section comprises the tubular assembly according to the present invention for circulating and heating manure.

Referring to the drawing, shown at 1 is the wall of a cylindrical silo, made of curved sheets, and placed on a concrete floor 6, as used, appropriately modified as a fodder silo on, for example, a dairy farm. Used within the framework of the apparatus according to the present invention, the bottom part of the silo is arranged as a manure fermenting container 2 which can be filled with manure up to level 3. From a manure store not shown, the manure is supplied to container 2 by means of a pump not shown through conduit 4, and the fermented manure can be discharged through pipe 5.

Designated by 7 and 8 are the supply and discharge conduits of a heat exchanger mounted in the manure fermenting container, which heat exchanger will be described hereinafter and by means of which the manure is kept at a temperature of approximately 30°C, which is the optimum temperature for anaerobic fermentation.

The gas produced during the fermentation of the manure rises and is collected in a gas buffer section 10. The gas buffer section 10 comprises a roof 30 which along the circumference thereof is in a leak-proof way secured to the silo wall 1 via a flexible wall 11, which is suitably made of a polyvinyl chloride material reinforced with canvas, being a material of great strength. The leak-proof connection of the flexible wall 11 to the silo wall 1 can be suitably produced by securing the flexible wall 11 along its circumference to the silo wall by means of uniformly spaced bolt connections, preferably using a thrust ring.

The roof 30 of the gas buffer section 10 is of frusto-conical form with a top surface 31 and wall 32. At the base end of the cone, a concrete disk 33 is connected to it as a weighting body. The weight of disk 33 is selected to suit the desired gas pressure of the biogas in the gas buffer section 10 and can easily be made such that a gas pressure will be produced which is suitable for use of the biogas as a fuel for domestic cooking appliances or even a still higher pressure which is sufficient to operate an internal combustion engine with it. Designated by 9 is a gas discharge pipe which is connected to the gas buffer section 10 and by means of which the produced biogas can be withdrawn from the stock and, for example, supplied to an combustion engine or to the consumer. Owing to the low location of the heavy disk 33 relative to roof 30, and in combination with the conical wall 32 of the roof it is achieved that the pressure of the gas against the inside wall 32 and the weight of disk 33 cooperate in such a manner that roof 30 can rise and descend without being laterally supported, or in other words, the roof is displaceable in a self-stabilizing manner.

In its lowest position, roof 30 is supported through disk 33 on a supporting table 34 at a predetermined distance above the manure level to be maintained in the apparatus.

A dip pipe 35, open at both ends, and connected co-axially to roof 30, functions as a safety element. When, for one reason or another, the gas pressure in gas buffer holder 10 becomes too high, the upper end of pipe 35 can rise into the opening 36 of the silo wall, while at the same time its bottom end rises above the manure level 3, owing to which biogas can be bled off from the gas section holder 10 through pipe 35 to the atmosphere.

37 designates a tubular assembly, which can also be used as a heat exchanger. The tubular assembly with an end 42 extending diagonally downwardly, and from this end it is of double-walled construction over a given length to form a section functioning as a heat exchanger 38 with supply and discharge pipes 7 and 8 for heating fluid, for example water. Axially mounted within the tubular assembly is a propeller stirrer 41 which via a shaft not shown and via the open other end 39 is connected to an electric motor, not shown, mounted outside the silo. The sub-assembly of the electric motor and the shaft with the propeller stirrer 41 connected thereto is of such construction that it can be axially removed from the pump as a unit, should this be desired, for example, for cleaning purposes. Designated by 40 is a manure supply opening, through which when the propeller stirrer rotates manure is drawn in, whereafter, owing to the thrust action of stirrer 41, the manure flows back into the manure fermenting container 2 through the open bottom end 42.

For optimizing the heat balance it is ensured, in a manner not shown, that the fermented manure discharged from the apparatus through conduit 5 is in heat-exchange contact with the manure to be fermented, supplied to the apparatus through conduit 4.

If for one reason or another the manure fermenting tank should be emptied this can be realised by pumping the manure out of the tank through conduit 12, which normally is closed by a valve and is connected to a pump, both members not being shown in the drawing.

The apparatus according to the invention is fully commensurate with installations already in use in farming, which makes for technical simplification and hence economy. Naturally, the apparatus described above and shown in the accompanying drawings may be modified without departing from the scope of the invention.

**Claim**

A closed apparatus for preparing biogas from manure, comprising a wall member (1), a manure fermenting section (2), means (4) for supplying manure to and means (5) for discharging fermented manure from said manure fermenting section (2), and means (9) for discharging the biogas prepared from the apparatus, and the apparatus further comprising means (7, 8) for heating and means (41) for stirring the manure both means being combined in a tubular assembly (37) through which a fluid can flow and which is double-walled over at least part of its length to allow circulation of a heating fluid between the double walls and through supply and discharge conduits (7, 8) connected thereto, characterized in that the tubular assembly (37) is arranged so that it extends diagonally downward through the wall member (1) of the apparatus into the manure, and a manure supply opening (40) is provided at a single-walled portion of the tubular assembly which is located between the double-walled portion (38) and the wall member of the apparatus, towards the upper end (39) of the tubular assembly and the stirring member, mounted on a shaft, is inserted through the upper end into the tubular assembly and is located below the manure supply opening (40).

## Revendication

Un appareil fermé pour préparer du biogaz de fumier, comprenant un organe de paroi (1), une section de fermentation de fumier (2), moyens (4) pour approvisionner du fumier à ladite section de fermentation de fumier (2) et des moyens (5) pour décharger du fumier fermenté de ladite section (2), ainsi que des moyens (9) pour décharger le biogaz préparé de l'appareil, ledit appareil comprenant également de moyens (7 8) pour échauffer et des moyens (41) pour brasser le fumier, ces deux moyens étant combinés dans une unité tubulaire (37) permettant l'écoulement d'un fluide à travers et qui est à double paroi sur au moins une partie de sa longueur pour permettre la circulation d'un fluide d'échauffement entre les doubles parois et à travers des conduits d'approvisionnement et de décharge (7, 8) y joints, caractérisé en ce que l'unité tubulaire (37) est aménagée de telle sorte qu'elle s'étend diagonalement vers le bas à travers l'organe de paroi (1) de appareil jusque dans le fumier, et qu'on a prévu une ouverture d'approvisionnement de fumier (40) dans une portion à simple paroi de l'unité tubulaire qui est située entre la portion à double paroi (38) et l'organe de paroi de l'appareil, vers le bout supérieur (39) de l'unité tubulaire et l'organe de brassage, monté sur un arbre, est introduit à travers le bout supérieur jusque dans l'unité tubulaire et est situé au-dessous de l'ouverture d'approvisionnement de fumier (40).

## Patentanspruch

Geschlossener Apparat zur Herstellung von Biogas aus Mist, mit einer Wandung (1), mit einem Mistgärteil (2), mit einem Mittel (4) zum Zuführen von Mist zum und mit einem Mittel (4) zum Entnehmen von gegorenem Mist von Mistgärteil (2) und mit einem Mittel (9) zum Entnehmen von im Apparat hergestelltem Biogas, ferner mit einem Mittel (7, 8) zum Erwärmen und einem Mittel (41) zum Umwälzen des Mistes, wobei beide Mittel in einer Rohrförmigen Anordnung (37) kombiniert sind, durch welche eine Flüssigkeit strömen kann und die über mindestens einen Teil ihrer Länge doppelwandig ist, um die Zirkulation einer Heizflüssigkeit zwischen den Doppelwänden und den damit verbundenen Zu- und Entnahmeleitungen (7, 8) zu ermöglichen, dadurch gekennzeichnet, daß die rohreförmige Anordnung (37) derart angeordnet ist, daß sie sich diagonal nach unten Durch die Wandung (1) des Apparates in den Mist hinein erstreckt, und daß eine Mistzuführöffnung (40) an einem Einfachwandbereich der Rohrförmigen Anordnung, der zwischen dem Doppelwandbereich (38) und der Wandung des Apparates zum oberen Ende (39) der rohrförmigen Anordnung hin angeordnet ist, vorgesehen ist, und daß das Umwälzelement, das an einer Welle befestigt ist, durch das obere Ende in die rohrförmige Anordnung eingesetzt und unterhalb der Mistzuführöffnung (40) angeordnet ist.